# EUROPEAN PATENT SPECIFICATION

(11) **EP 3 752 488 B1**
(45) Date of publication and mention of the grant of the patent: **08.03.2023**
(21) Application number: 19706921.4
(22) Date of filing: 13.02.2019
(51) Int. Cl.: C07D 213/75, C07D 513/04

(54) **METHOD FOR PREPARING TERT-BUTYL N-((1R,2S,5S)-2-((2-((5-CHLOROPYRIDIN-2-YL)AMINO)-2-OXOACETYL)AMINO)-5-(DIMETHYLCARBAMOYL)CYCLOHEXYL)CARBAMATE**
VERFAHREN ZUR HERSTELLUNG VON TERT-BUTYL N-((1R,2S,5S)-2-((2-((5-CHLOROPYRIDIN-2-YL)AMINO)-2-OXOACETYL)AMINO)-5-(DIMETHYLCARBAMOYL)CYCLOHEXYL)CARBAMAT
PROCÉDÉ DE PRÉPARATION DE N-((1R,2S,5S)-2-((2-((5-CHLOROPYRIDIN-2-YL)AMINO)-2-OXOACÉTYL)AMINO)-5-(DIMÉTHYLCARBAMOYL)CYCLOHEXYL)CARBAMATE TERT-BUTYLE

(30) Priority: 14.02.2018 ES 201830131
(43) Date of publication of application: 23.12.2020
(73) Proprietor: Moehs Ibérica, S.L., 08191 Rubí-Barcelona (ES)
(72) Inventor: GARCÍA GARCÍA, Elena, 08191 Rubí - Barcelona (ES); DOBARRO RODRÍGUEZ, Alicia, 08191 Rubí - Barcelona (ES)
(74) Representative: ABG Intellectual Property Law, S.L.
(86) International application number: PCT/EP2019/053484
(87) International publication number: WO 2019/158550

(56) References cited:
- EP-A1- 1 925 611
- EP-A1- 2 407 450
- EP-A1- 2 407 457
- EP-A1- 2 589 590
- WO-A1-2018/011823
- US-A1- 2017 260 138

## Description

### Field of the Invention

The present invention relates to a method for preparing *tert*-butyl N-((1R,2S,5S)-2-((2-((5-chloropyridin-2-yl)amino)-2-oxoacetyl)amino)-5-(dimethylcarbamoyl)cyclohexyl)carbamate of formula (I) 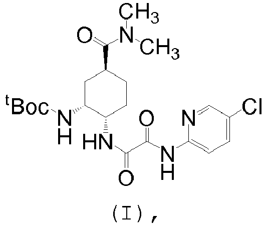 which is an intermediate product useful in the synthesis of the drug, known as N'-(5-chloropyridin-2-yl)-N-[(1S,2R,4S)-4-(dimethylcarbamoyl)-2-[(5-methyl-6,7-dihydro-4H-[1,3]thiazolo [5,4-c]pyridine-2-carbonyl)amino]cyclohexyl]oxamide or as N-(5-chloropyridin-2-yl)-N'-((1S,2R,4S)-4-(dimethylcarbamoyl)-2-((5-methyl-4,5,6,7-tetrahydrothiazol[5,4-c]pyridin-2-carbonyl) amino) cyclohexyl) oxamide, best known as Edoxaban.

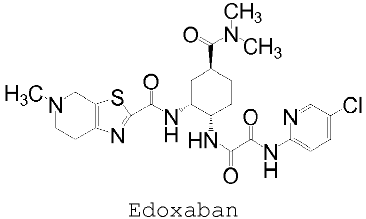

### Background of the Invention

International application WO2007/032498A describes optically active amines as intermediate products in the synthesis of the drug Edoxaban. Edoxaban is a direct inhibitor of coagulation factor Xa and is sold under the trade name Lixiana^{®} as an oral anticoagulant.

The compound of formula (I), *tert*-butyl N-((1R,2S,5S)-2-((2-((5-chloropyridin-2-yl)amino)-2-oxoacetyl)amino)-5-(dimethylcarbamoyl)cyclohexyl)carbamate, is a highly important intermediate product in the synthesis of Edoxaban. The state of the art describes the synthesis of said intermediate product from the *tert*-butyl N-((1R,2S,5S)-2-amino-5-(dimethylcarbamoyl)cyclohexyl)carbamate oxalate salt of formula (A·oxalate) and from the ethyl 2-((5-chloropyridin-2-yl)amino)-2-oxoacetate hydrochloride salt of formula (B·HCl) (WO2007/032498A1, WO2010/104106A1, WO2012/002538A1, and also WO2012/017932A1).

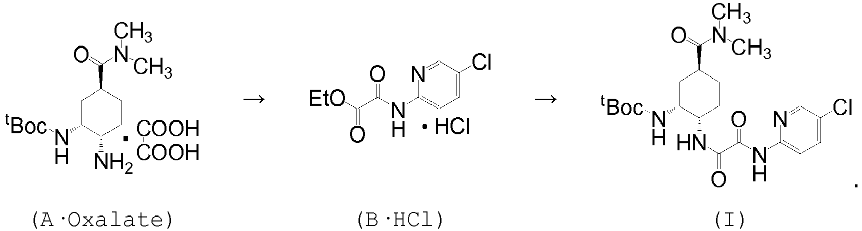

According to the method described in the mentioned documents, the oxalate salt of formula (A·Oxalate) is reacted with the hydrochloride salt of formula (B·HCl) at 60 °C in acetonitrile in the presence of triethylamine (4.6 equivalents). However, said method is not satisfactory on an industrial level not only because it causes the solidification of the reaction mass, leading to 85% production yields for the product of formula (I), but also because it requires large amounts of the base, triethylamine, with a triethylamine:acetonitrile proportion by volume of 1:3.3.

Patent document WO2010/104078A1 attempts to solve these problems using triethylamine in two different steps and in lower amounts. With these modifications, the production yield for the compound of formula (I) increases up to 93%. However, this method requires constant input to fine-tune the control of the reaction steps, the order of addition of the reagents, as well as their amounts, and does not satisfactorily solve the problem of the increase in viscosity of the reaction medium, which complicates stirring and is thus rather undesirable on industrial scales.

Document WO 2018/011823 A1 describes the synthesis of the intermediate of formula (I) from the camphorsulfonate salt of the compound of formulas (A) and (B).

Nevertheless, it is still highly relevant to provide a simple method for the synthesis of the compound of formula (I), or a salt or solvate thereof, with high purity and yield, which does not lead to a dramatic increase in the viscosity of the reaction medium and which can therefore be carried out in a conventional reactor at an industrial scale.

In this sense, the inventors have developed a new synthesis method, described in the present application and defined by the claims, for obtaining the compound of formula (I), or a salt or solvate thereof, which solves the problems described above.

### Brief Description of the Invention

The present invention relates to a new method for the synthesis of the product of formula (I) without increasing the viscosity of the reaction medium, and therefore with higher reaction yields and higher purity than the methods described in the state of the art. The gist of the invention lies in using the reagents of formula (A) and (B) in their neutral forms. This is a surprising discovery since the state of the art does not suggest at any time, and much less describe, that the use of neutral forms of the reagents would lead to a substantial improvement in the synthesis of the compound of formula (I).

Therefore, a first aspect of the invention relates to a method for preparing *tert*-butyl N-((1R,2S,5S)-2-((2-((5-chloropyridin-2-yl)amino)-2-oxoacetyl)amino)-5-(dimethylcarbamoyl)cyclohexyl)carbamate of formula (I) 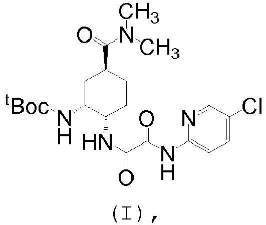 or a salt or solvate thereof, characterized in that it comprises the steps of:
a) mixing *tert*-butyl N-((1R,2S,5S)-2-amino-5-(dimethylcarbamoyl)cyclohexyl)carbamate of formula (A), 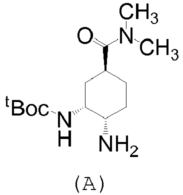 with ethyl 2-((5-chloropyridin-2-yl)amino)-2-oxoacetate of formula (B), 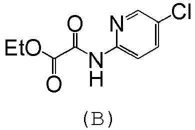 in an organic solvent;
b) mixing a base with the resulting mixture from step (a); and
c) stirring the mixture obtained in step (b).

The first aspect, also identified in the present application as the method of the invention, allows obtaining in a simple manner, with high yield and/or purity, the compound of formula (I) or a salt or solvate thereof. The compound of formula (I) is highly relevant in the synthesis of the Edoxaban as it is its direct precursor.

### Brief Description of the Drawings

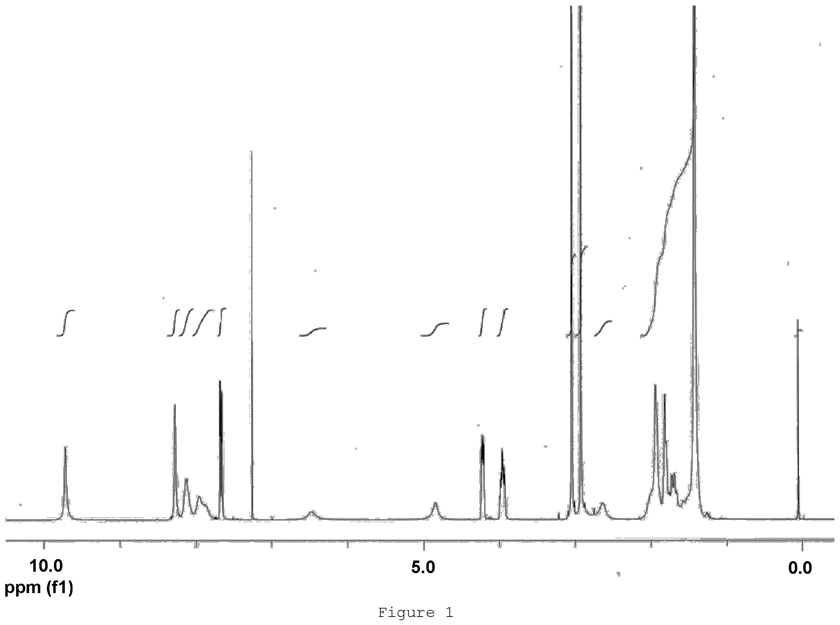

Figure 1. ¹H-NMR (CDCl₃, 400 MHz) of *tert*-butyl N-((1R,2S,5S)-2-((2-((5-chloropyridin-2-yl)amino)-2-oxoacetyl)amino)-5-(dimethylcarbamoyl)cyclohexyl)carbamate of formula (I).

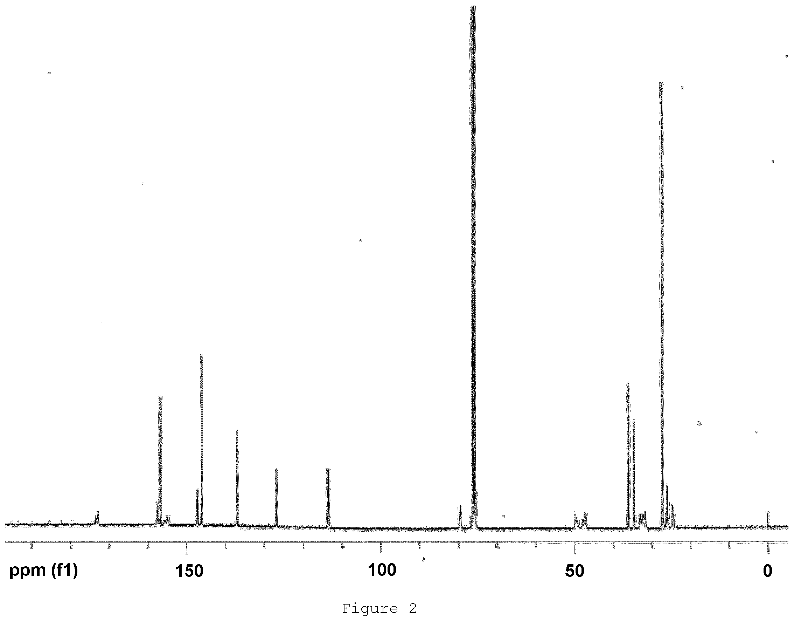

Figure 2. ¹³C-NMR (CDCl₃, 100 MHz) of tert-butyl N-((1R,2S,5S)-2-((2-((5-chloropyridin-2-yl)amino)-2-oxoacetyl)amino)-5-(dimethylcarbamoyl)cyclohexyl)carbamate of formula (I).

### Detailed Description of the Invention

The present invention lies in the discovery that the synthesis of the compound of formula (I) described in the state of the art is much simpler and provides better yields and purities when the compounds of formula (A) and (B), in their neutral state (i.e., they are not salts), are used as starting materials. Unlike the method described in the state of the art, the synthesis of the compound of formula (I) of the present invention does not require careful control of the order of addition of the reagents, nor does it lead to a dense reaction mixture which is difficult to stir. These advantages are made evident from the examples herein described.

The first aspect of the invention, also identified in the present application as the method of the invention, relates to a method for preparing tert-butyl N-((1R,2S,5S)-2-((2-((5-chloropyridin-2-yl)amino)-2-oxoacetyl)amino)-5-(dimethylcarbamoyl)cyclohexyl)carbamate of formula (I) 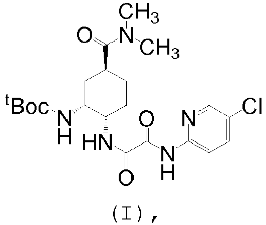 or a salt or solvate thereof, characterized in that it comprises the steps of:
a) mixing *tert* butyl N-((1R,2S,5S)-2-amino-5-(dimethylcarbamoyl)cyclohexyl)carbamate of formula (A), 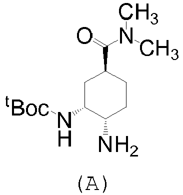 with ethyl 2-((5-chloropyridin-2-yl)amino)-2-oxoacetate of formula (B) 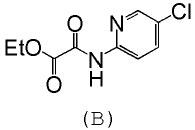 in an organic solvent;
b) mixing a base with the resulting mixture from step (a); and
c) stirring the mixture obtained in step (b).

In the present application and in the claims, the term "*^{t}*Boc" represents the tert-butyloxycarbonyl group.

The term salt refers to a salt prepared from a conjugate acid or base of the compound of formula (I) or (II) and can be derived from inorganic or organic acids including, without being limited to, hydrochloric, hydrobromic, sulfuric, phosphoric, nitric, methanesulfonic, ethanesulfonic, benzenesulfonic, p-toluenesulfonic, camphorsulfonic, 1,5-naphthalenedisulfonic acids, formic, acetic, benzoic, malonic, malic, citric, fumaric, gluconic, glycolic, glutamic, lactic, maleic, L-tartaric, oxalic, succinic. Preferably, the acid is p-toluenesulfonic acid or hydrochloric acid.

The term solvate refers to solid molecular compounds that have incorporated a crystallization solvent molecule in their crystal lattice. When the solvent incorporated in the solvate is water, the solvate is called hydrate. All the solvates are formed with stoichiometric or non-stoichiometric proportions of the compound and the crystallization solvent. The solvates can exhibit polymorphism, i.e., they can exist in more than one polymorphic form. In a particular embodiment of the present invention, the compound of formula (I) is a solvate of a solvent selected from acetonitrile, water, or alcohol.

### Reagents of formula (A) and (B)

Throughout the present specification, the compounds of formula (A) and (B) may also be referred to as reagents (A) and (B), or simply (A) and (B). The method of the present invention has the enormous advantage of being simpler than that described in the state of the art because it allows the mixture of the reagents of formula (A) and (B) in their neutral form, without having to take into account what the order of reagent addition is and can be performed in a single step.

In a particular embodiment, the molar ratio between the compounds of formula (A) and (B), in step (a) of the method of the invention, is from 1:0.5 to 1:2, preferably from 1:0.95 to 1:1.5, more preferably from 1:1.05 to 1:1.35, and even more preferably 1:1.05. In the context of the present invention, a molar ratio between the compounds of formula (A) and (B) of 1:1.05 means that for every mol of compound of formula (A) there are 1.05 mol of the compound of formula (B).

In another particular embodiment, the mass concentration of each of the compounds of formula (A) and (B) is from 1 to 40%, preferably from 15 to 40%, expressed in grams of solute per milliliter of solution, multiplied by 100. In other words, a concentration of from 1 to 40% means from 10 grams to 400 grams for each liter of solvent. In this sense, 800 grams of the compound of formula (A) in 4 L of organic solvent correspond to a mass concentration of 20%.

In a particular embodiment, the mass concentration of any one of the compounds of formula (A) or (B) is less than 40%, less than 35%, less than 30%, less than 25%, or less than 20%. Preferably, the mass concentration of any one of the compounds of formula (A) or (B) is less than 40°.

In another particular embodiment, the mass concentration of any one of the compounds of formula (A) or (B) is greater than 10%, greater than 15%, greater than 20%, greater than 25%, greater than 30%, or greater than 35%. Preferably, the mass concentration of any one of the compounds of formula (A) or (B) is greater than 15%.

### Organic solvent

The reaction can be carried out in the presence of an organic solvent selected from cyclic ether (for example 1,4-dioxane, tetrahydrofuran, methyltetrahydrofuran), linear or branched ketones (for example, methylisobutylketone or methylethylketone), amide (for example dimethylformamide or dimethylacetamide), alcohol (for example, methanol, ethanol, propanol, isopropanol, butanol, isobutanol), nitrile (for example, acetonitrile), or mixtures thereof.

In a preferred embodiment, the organic solvent is selected from the group consisting of acetonitrile, N,N-dimethylformamide, linear or branched C₁-C₄ alkyl alcohol, or mixtures thereof.

### Base

The step of mixing a base can be done simply by addition of the base to the mixture of the reagents of formula (A) and (B).

The base used in the reaction can be selected from tertiary amines and aromatic amines, as well as mixtures thereof.

In the context of the method of the invention, the term "aromatic amines" is used to designate aromatic compounds with one or more rings comprising 4-9 carbon atoms and 1-2 nitrogen atoms. Examples of aromatic amines are pyridine, methylpyridine, dimethylpyridine, dibutylmethylpyridine, and dimethylaminopyridine.

In the context of the method of the invention, the term "tertiary amines" is used to designate amines in which a nitrogen atom is linked to three identical or different C1 to C4 alkyl radicals. Examples of tertiary amines are trimethyl-, triethyl-, tripropyl-, tributylamine or diisopropylethylamine.

In a preferred embodiment, the base is a tertiary amine, aromatic amine, and mixtures thereof.

In a particular embodiment, the base is a tertiary amine substituted with C₁-C₄ alkyl or mixtures thereof. Preferably, the base used in the reaction is a non-nucleophilic base, for example a tertiary amine substituted with C₁-C₄ alkyl.

In a preferred embodiment, the base used is selected from diisopropylethylamine, triethylamine, or a mixture thereof, preferably triethylamine.

In a particular embodiment, the base is added to the mixture of step (a), wherein the mixture of step (a) was previously heated. Preferably, in the method of the invention, the base is added in a controlled manner, such that the addition of the base does not cause a change in the reaction temperature. One skilled in the art will know how to carry out the step of adding the base in a controlled manner. A non-limiting example would be, for example, measuring the temperature of the reaction to control the rate of addition of the base such that said addition does not cause a significant increase (or decrease) in temperature.

In a particular embodiment, the total amount of base added in step (b) corresponds to a molar ratio between the compound of formula (A) and the base of from 1:0.1 to 1:2.5, preferably of from 1:0.25 to 1:2.5. This means that in a particular embodiment of the method of the invention, in step (b) there are between 0.25 and 2.5 mol of base for every mol of compound of formula (A). Preferably, the molar ratio between the compound of formula (A) and the base is 1:0.5. In another preferred embodiment, the molar ratio between the compound of formula (A) and the base is from 1:0.1 to 1:0.5.

### Reaction medium and experimental conditions

The method of the present invention provides a simple manner of obtaining a compound of formula (I), in which some of the advantages of the method with respect to the state of the art lies in the fact that the reaction mixture is workable in the sense that it does not form a paste that is difficult to stir.

In the context of the method of the invention, the term "mixture", when applied to the result of mixing one compound with another, must be understood as a suspension. Therefore, in a particular embodiment, step (b) leads to a suspension. In a particular embodiment, step (c) leads to a suspension.

As a result of the experimental conditions of the method of the invention, the mixture obtained in step (c) has a lower viscosity than that of the reaction medium of the state of the art. For this reason, the method of the invention allows easy stirring of the reaction medium, which allows simplifying the method not only regarding stirring but also in working the crude reaction product, which entails an increase in purity and/or yield. Up until the present invention, the methods of the state of the art only led to dense and difficult to stir mixtures.

Throughout the present specification, the temperature is indicated in Celsius degrees (°C) and temperature values must be understood as being associated with an experimental error of ± 2 °C.

In a particular embodiment of the method of the invention, the mixture of the reagents of formula (A) and (B) in the step (a) is carried out at room temperature, with said temperature being comprised between 15 and 30 °C, preferably between 20 and 25 °C. Alternatively, after mixing the reagents of formula (A) and (B), the resulting mixture is heated at a temperature comprised between 30 °C and 200 °C. In a preferred embodiment, step (a) comprises the additional step of heating the mixture at a temperature comprised between 30 °C and 100 °C, preferably 30 °C to 70 °C.

If the organic solvent, because of its boiling temperature, does not allow heating the mixture obtained in step (a) at a temperature within the range comprised between 30 °C and 200 °C, then one skilled in the art will understand that the reaction temperature will be the boiling temperature of the solvent, or alternatively, it will be necessary to increase the reaction pressure. The two scenarios are contemplated by the method of the present invention.

In a preferred embodiment, step (c) further comprises the step of increasing the temperature of the mixture obtained in step (b) between 10 °C and 50 °C. This means that after adding the base to a mixture comprising compounds (A) and (B), the reaction temperature is increased, in said preferred embodiment, by between 10 and 50 °C with respect to the previous temperature. In another preferred embodiment, the base is added in step (b) to an organic solvent comprising the compounds of formula (A) and (B) which was heated at a temperature between 30 °C and 70 °C in step (a), and, in step (c), the temperature is further increased by 10 to 50 °C for reacting under stirring.

One skilled in the art will understand that said step (a) may or may not be carried out by means of stirring. In the field of the art, except in exceptional situations, steps such as step (a) are carried out under stirring. Therefore, in a particular embodiment, step (a) is carried out under stirring. In another particular embodiment, the method of the invention is a method which is carried out under stirring during steps (a), (b), and (c) .

Since a low-viscosity reaction medium is achieved in the method of the present invention, the stirring does not require any special technique so it may achieved by magnetic stirring, ultrasonic-assisted stirring, orbital shakers, vortex, paddle mixer, or other means known in the field of the art, provided that they allow stirring of the reaction mixture such that said mixture is rendered homogeneous.

In a particular embodiment, the step of stirring of step (c) of the method of the invention takes at least 1 hour. In another particular embodiment, said step of stirring takes at least 3 hours. In yet another particular embodiment, the step of stirring is carried out between 1 and 10 hours. The reaction time will depend on the temperature and on the amounts of reagents (A) and (B), but one skilled in the art will have no difficulty determining the best time to stop the reaction, knowing, as a result of the present application, that the yield of the product of formula (I) can be calculated from, for example, HPLC chromatography, as is demonstrated in the examples of the application. Preferably, the reaction is carried out for a time interval of at least 1 hour, at least 2 hours, or at least 3 hours. In a preferred embodiment, the step of stirring of step (c) of the method of the invention is carried out between 1 hour and 10 hours, more preferably between 3 hours and 8 hours.

### Optional additional steps

Steps (a), (b), and (c), as defined in the claims, are sufficient for preparing the tert-butyl N-((1R,2S,5S)-2-((2-((5-chloropyridin-2-yl)amino)-2-oxoacetyl)amino)-5-(dimethylcarbamoyl)cyclohexyl)carbamate compound of formula (I). Nevertheless, the method of the present invention also contemplates the optional additional step, which allows isolating the compound of formula (I).

Therefore, in a particular embodiment, the method of the invention comprises the additional steps of (d) decreasing the reaction temperature to a final temperature between -5 °C and 10 °C, and (e) subsequently filtering the compound of formula (I).

Optionally, step (d) further comprises the initial steps of decreasing the reaction temperature to a temperature of between 15 and 30 °C and adding water to the organic solvent, before the step of lowering the temperature to a final temperature of between -5 °C and 10 °C. In a preferred embodiment of said optional step, the water is added in a water:solvent proportion (v/v) of 0.2:1 to 2:1. More preferably, the water:solvent proportion is 0.5:1 to 0.8:1 (v/v).

### Tert-butyl N-((1R,2S,5S)-2-((2-((5-chloropyridin-2-yl)amino)-2-oxoacetyl)amino)-5-(dimethylcarbamoyl)cyclohexyl)carbamate of formula (I)

The compound of formula (I) is a highly relevant precursor in the synthesis of the compound of formula (II), Edoxaban, since it is its direct precursor. The method of the invention provides a very simple, easy, and therefore cost-effective manner of obtaining the compound of formula (I), which avoids the problems of the state of the art, such as the increase in viscosity of the medium (with subsequent stirring difficulty).

The compound of formula (I) directly obtainable according to the method of the invention can be used directly without isolation in the preparation of the compound of formula (II), i.e., after step (c) of the method of the invention.

Therefore, in a particular embodiment, the method of the present invention comprises the additional step of transforming the compound of formula (I), or a salt or solvate thereof, in N-(5-chloropyridin-2-yl)-N'-((1S,2R,4S)-4-(dimethylcarbamoyl)-2-((5-methyl-4,5,6,7-tetrahydrothiazol[5,4-c]pyridin-2-carbonyl)amino)cyclohexyl)oxamide of formula (II) 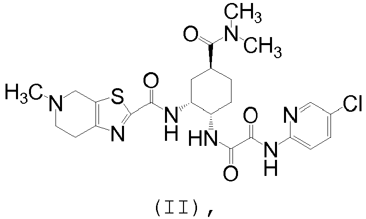 or a salt or solvate thereof.

The essentials necessary for obtaining the compound of formula (II) from the compound of formula (I) can be found in the state of the art, as well as in Example 3 of the present application.

### Examples

The purity of the obtained products was analyzed by means of high performance liquid chromatography in a Waters Alliance equipment, provided with a variable wave detector and a thermostat-controlled oven for the column. The experimental conditions for obtaining a chromatogram were:
Column: BEH C18 3.0 x 50 mm x 1.7 um
Mobile phase A: 5 mM HCO₂NH₄ in water
Mobile phase B: Acetonitrile
Flow rate: 0.5 mL/min
Column temp.: 40 °C
1 µL injection vol.
Detection wavelength: 290/210 nm
Sample solvent.: Acetonitrile/Water (50:50)
Concentration: 1 mg/mL
t(minutes):
   0: 95% Phase A
   0.5: 95° Phase A
   9: 10% Phase A
   10: 10% Phase A
   11: 95% Phase A
   12: 95% Phase A

The *tert*-butyl N-((1R,2S,5S)-2-amino-5-(dimethylcarbamoyl)cyclohexyl)carbamate compound can be obtained as described in Reference Example 144 and preceding examples of document EP 1 405 852 A1.

The ethyl 2-((5-chloropyridin-2-yl)amino)-2-oxoacetate compound can be obtained as described in Reference Example 243 of EP 1 405 852 A1.

The 5-methyl-6,7-dihydro-4H-thiazol[5,4-c]pyridin-2-carboxylic acid hydrochloride salt can be obtained by means of other methods such as those described in document EP 1 683 800 A.

### Example 1. Synthesis of tert-butyl N-((1R,2S,5S)-2-((2-((5-chloropyridin-2-yl)amino)-2-oxoacetyl)amino)-5-(dimethylcarbamoyl)cyclohexyl)carbamate of formula (I)

800 g (2.80 mol) of tert-butyl N-((1R,2S,5S)-2-amino-5-(dimethylcarbamoyl)cyclohexyl)carbamate (of formula (A)) and 673 g (2.94 mol, 1.05 molar eq.) of ethyl 2-((5-chloropyridin-2-yl)amino)-2-oxoacetate (of formula (B)) were mixed with 4 L of acetonitrile. The resulting mixture was heated to a temperature of about 50 °C and, maintaining the indicated temperature, 269 mL (195 g, 1.4 mol, 0.5 molar eq.) of triethylamine were slowly added. The resulting mixture was heated at a temperature of about 60 °C and kept under stirring for 8 hours at the indicated temperature.

The reaction mass was then cooled at a temperature of between 0 and 5 °C and filtered. The resulting solid was washed three times with 800 mL of water each, and subsequently dried. 1210 g (yield of 92.2%) of a white solid corresponding to *tert-*butyl N-((1R,2S,5S)-2-((2-((5-chloropyridin-2-yl)amino)-2-oxoacetyl)amino)-5-(dimethylcarbamoyl)cyclohexyl)carbamate were thereby obtained. Product purity was analyzed by means of HPLC (t^{retention} = 5.73 min), obtaining a value of 99.35%.

¹H-NMR (CDCl₃, 400 MHz) 5(ppm): 9.72 (1H, s), 8.28 (1H, s), 8.13 (1H, s), 7.80-8.02 (1H, s broad), 7.67 (1H, dd), 6.47 (0.4H, s broad), 4.85 (0.6H, s broad), 4.22 (1H, m), 3.96 (1S, m), 3.05 (3H, s), 2.93 (3H, s), 2.64 (1H, s broad), 1.64-2.10 (5H, m), 1.25-1.60, (11H, s).

¹³C-NMR (CDCl₃, 100 MHz) δ(ppm): 173.86, 158.61, 157.80, 148.15, 147.06, 137.96, 127.82, 114.43, 80.48, 50.88, 50.35, 48.84, 48.28, 37.06, 35.67, 33.99, 33.25, 32.69, 28.23, 27.03, 25.62.

### Example 2. Synthesis of tert-butyl N-((1R,2S,5S)-2-((2-((5-chloropyridin-2-yl)amino)-2-oxoacetyl)amino)-5-(dimethylcarbamoyl)cyclohexyl)carbamate of formula (I)

80 g (2.80 mol) of tert-butyl N-((1R,2S,5S)-2-amino-5-(dimethylcarbamoyl)cyclohexyl)carbamate (of formula (A)) and 67.3 g (0.294 mol, 1.05 molar eq.) of ethyl 2-((5-chloropyridin-2-yl)amino)-2-oxoacetate (of formula (B)) were mixed with 400 mL of acetonitrile. The resulting mixture was heated to a temperature of about 50 °C and, while maintaining the indicated temperature, 27 mL (19.5 g, 1.4 mol, 0.5 molar eq.) of triethylamine were slowly added. The resulting mixture was heated at the temperature of about 60 °C and kept under stirring for 7 hours at the indicated temperature.

The reaction mass was then cooled at the temperature of between 20 and 25 °C and 320 mL of water were added. The reaction mass was cooled at a temperature of between 0 and 5 °C and filtered. The resulting solid was washed three times with 80 mL of water each, and subsequently dried. 122.0 g (yield of 93.0%) of a white solid corresponding to tert-butyl N-((1R,2S,5S)-2-((2-((5-chloropyridin-2-yl)amino)-2-oxoacetyl)amino)-5-(dimethylcarbamoyl)cyclohexyl)carbamate were thereby obtained. Product purity was analyzed by means of HPLC, obtaining a value of 98.96%.

### Example 3. Synthesis of N-(5-chloropyridin-2-yl)-N'-((1S,2R,4S)-4-(dimethylcarbamoyl)-2-((5-methyl-4,5,6,7-tetrahydrothiazol[5,4-c]pyridin-2-carbonyl)amino)cyclo hexyl)oxamide of formula (II)

1000 g (2.14 mol) of tert-butyl N-((1R,2S,5S)-2-((2-((5-chloropyridin-2-yl)amino)-2-oxoacetyl)amino)-5-(dimethylcarbamoyl)cyclohexyl)carbamate (of formula (I)), obtained according to any one of the methods described in Examples 1 or 2, were mixed with 12.5 L of acetonitrile. The temperature of the resulting mixture was maintained between 20 and 25 °C during the subsequent addition of 278 mL (411.4 g, 4.28 mol, 2 molar eq.) of methanesulfonic acid. The resulting mixture was heated at a temperature of about 60 °C and kept under stirring for 3 hours at the indicated temperature.

The reaction mixture was then cooled at a temperature of between 0 and 5 °C and 625 mL (453.7 g, 4.49 mol, 2.10 molar eq.) of triethylamine were slowly added, maintaining the temperature below 5 °C. The mass reaction temperature was controlled to keep it between 20 and 25 °C, and then 527 g (2.24 mol, 1.05 molar eq.) of the 5-methyl-4,5,6,7-tetrahydrothiazol[5,4-c]pyridin-2-carboxylic acid hydrochloride salt, 30.4 g of OximaPure^{®} (cyano(hydroxyimino) ethyl acetate) (0.214 mol, 0.1 molar eq.), and 384 mL (313 g, 2.48 mol, 1.16 molar eq.) of diisopropylcarbodiimide were added, while keeping the temperature between 20 and 25 °C. The reaction mixture was kept under stirring for 5 hours in said temperature range.

2 L of triethylamine were then added, keeping the temperature between 20 and 25 °C, and subsequently 5 L of water were added in the same temperature range. The resulting mixture was cooled at a temperature of about 3 °C and filtered. The resulting solid was washed three times 1 L of water each, and subsequently dried. 1129.0 g (yield of 96.4%) of a white solid corresponding to N-(5-chloropyridin-2-yl)-N'-((1S,2R,4S)-4-(dimethylcarbamoyl)-2-((5-methyl-4,5,6,7-tetrahydrothiazol[5,4-c]pyridin-2-carbonyl)amino)cyclohexyl)oxamide were thereby obtained. Product purity was analyzed by means of HPLC, obtaining a value of 98.19%.

### Comparative example 1. Synthesis of tert-butyl N-((1R,2S,5S)-2-((2-((5-chloropyridin-2-yl)amino)-2-oxoacetyl)amino)-5-(dimethylcarbamoyl)cyclohexyl)carbamate of formula (I) according to the method disclosed in Reference Example 1 of EP 1925611 A1 (WO 2007/032498 A1)

5 g of the tert-butyl N-((1R,2S,5S)-2-amino-5-(dimethylcarbamoyl)cyclohexyl)carbamate oxalate salt (A·oxalate) were suspended in 27 mL of acetonitrile. The obtained mixture was heated at a temperature of about 60 °C and 8.5 mL of triethylamine were slowly added to the obtained mixture, and subsequently 4.2 g of ethyl 2-((5-chloropyridin-2-yl)amino)-2-oxoacetate hydrochloride salt (B·HCl) were added. The resulting mixture was kept under stirring for 6 hours at a temperature of about 60 °C and then for 16 hours at a temperature between 20 and 25 °C.

30 mL of water were then added. The resulting mixture was cooled at a temperature of about 10 °C, kept under stirring for 1 hour and 30 minutes at said temperature, and finally filtered. The resulting solid was washed with 20 mL of water and subsequently dried. 5.4 g (yield of 87.1%) of a white solid corresponding to tert-butyl N-((1R,2S,5S)-2-((2-((5-chloropyridin-2-yl)amino)-2-oxoacetyl)amino)-5-(dimethylcarbamoyl)cyclohexyl)carbamate were thereby obtained. Product purity was analyzed by means of HPLC, obtaining a value of 98.29%.

### Comparative example 2. Synthesis of tert-butyl N-((1R,2S,5S)-2-((2-((5-chloropyridin-2-yl)amino)-2-oxoacetyl)amino)-5-(dimethylcarbamoyl)cyclohexyl)carbamate of formula (I) according to the method disclosed in Example 1 of EP 2407457 B1 (WO 2010/104078 A1)

16.96 g of the 2-((5-chloropyridin-2-yl)amino)-2-oxoacetate ethyl hydrochloride salt (B·HCl) were suspended in 100 mL of acetonitrile. 6.35 g of triethylamine were slowly added to the obtained mixture, and subsequently 20 g of *tert*-butyl N-((1R,2S,5S)-2-amino-5-(dimethylcarbamoyl)cyclohexyl) carbamate oxalate salt (A·oxalate) were added, maintaining a temperature of about 10 °C. The resulting mixture was heated to a temperature of about 60 °C, and 21.8 g of triethylamine were slowly added. The resulting mixture was heated at a temperature of about 70 °C and kept under stirring for 7 hours at the indicated temperature.

The reaction mass was then cooled at a temperature of about 25 °C and 180 mL of water were added. The resulting mixture was cooled at a temperature of about 10 °C and filtered. The resulting solid was washed with 100 mL of water and subsequently dried. 22.7 g (yield of 91.1%) of a white solid corresponding to tert-butyl N-((1R,2S,5S)-2-((2-((5-chloropyridin-2-yl)amino)-2-oxoacetyl)amino)-5-(dimethylcarbamoyl)cyclohexyl)carbamate were thereby obtained. Product purity was analyzed by means of HPLC, obtaining a value of 98.50%.

## Claims

1. A method for preparing tert-butyl N-((1R,2S,5S)-2-((2-((5-chloropyridin-2-yl)amino)-2-oxoacetyl)amino)-5-(dimethylcarbamoyl)cyclohexyl)carbamate of formula (I) 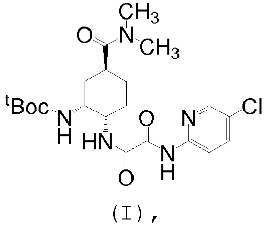 or a salt or solvate thereof, **characterized in that** it comprises the steps of:
a) mixing tert-butyl N-((1R,2S,5S)-2-amino-5-(dimethylcarbamoyl)cyclohexyl)carbamate of formula (A) 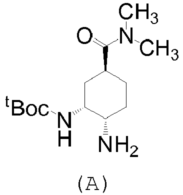 with ethyl 2-((5-chloropyridin-2-yl)amino)-2-oxoacetate of formula (B) 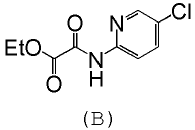 in an organic solvent;
b) mixing a base with the resulting mixture from step (a); and
c) stirring the mixture obtained in step (b).

2. The method according to claim 1, **characterized in that** the organic solvent is selected from the group consisting of acetonitrile, N,N-dimethylformamide, linear or branched C₁-C₄ alkyl alcohol, or mixtures thereof.

3. The method according to any one of claims 1 to 2, **characterized in that** the base is an amine selected from the group consisting of tertiary amines, aromatic amines and mixtures thereof.

4. The method according to any one of claims 1 to 3, **characterized in that** the base is diisopropylethylamine, triethylamine, or a mixture thereof, preferably triethylamine.

5. The method according to any one of claims 1 to 4, **characterized in that** step (a) further comprises the step of heating the mixture at a temperature comprised between 30 °C and 100 °C.

6. The method according to claim 5, **characterized in that** the step of heating the mixture is performed at a temperature comprised between 30 °C and 70 °C.

7. The method according to any one of claims 1 to 6, **characterized in that** step (c) further comprises the step of increasing the temperature of the mixture obtained in step (b) between 10 °C and 50 °C.

8. The method according to any one of claims 1 to 7, **characterized in that** the stirring of step (c) is carried out between 1 and 10 hours.

9. The method according to any one of claims 1 to 8, **characterized in that** it comprises the additional steps of (d) decreasing the reaction temperature to a final temperature between -5 °C and 10 °C and (e) subsequently filtering the product of formula (I).

10. The method according to any one of claims 1 to 9, **characterized in that** the molar ratio between the compounds of formula (A) and (B) is from 1:0.5 to 1:2, preferably from 1:0.95 to 1:1.5.

11. The method according to any one of claims 1 to 10, **characterized in that** the molar ratio between the compound of formula (A) and the base is from 1:0.1 to 1:2.5, preferably from 1:0.25 to 1:2.5.

12. The method according to any one of claims 1 to 11, **characterized in that** in step (a), the mass concentration of each of the compounds of formula (A) and (B) is from 15 to 40%, expressed in grams of solute per milliliter of solution multiplied by 100.

13. The method according to any one of claims 1 to 12, **characterized in that** it comprises the additional step of transforming the compound of formula (I), or a salt or solvate thereof, into N-(5-chloropyridin-2-yl)-N'-((1S,2R,4S)-4-(dimethylcarbamoyl)-2-((5-methyl-4,5,6,7-tetrahydrothiazol[5,4-c]pyridin-2-carbonyl)amino)cyclohexyl)oxamide of formula (II) 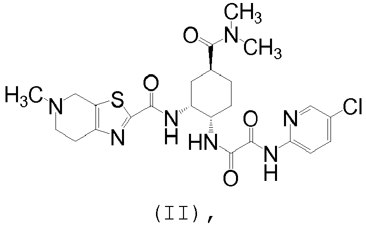 or a salt or solvate thereof.

## Patentansprüche

1. Verfahren zur Herstellung von *tert*-Butyl-*N*-((1R, 2S, 5S)-2-((2-((5-chlorpyridin-2-yl)amino)-2-oxoacetyl)amino)-5-(dimethylcarbamoyl)cyclohexyl)carbamat der Formel (I) 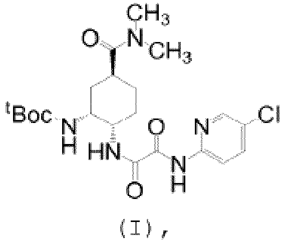 oder eines Salzes oder Solvats davon, **dadurch gekennzeichnet, dass** es die Schritte umfasst:
a) Mischen von *tert*-Butyl-*N*-((1R, 2S, 5S)-2-amino-5-(dimethylcarbamoyl)cyclohexyl)carbamat der Formel (A) 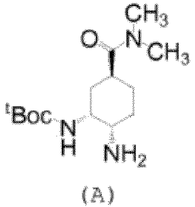 mit Ethyl-2-((5-Chlorpyridin-2-yl)amino)-2-oxoacetat der Formel (B) 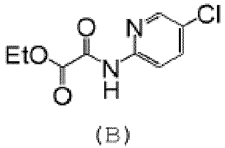 in einem organischen Lösungsmittel;
b) Mischen einer Base mit der resultierenden Mischung aus Schritt (a); und
c) Rühren der in Schritt (b) erhaltenen Mischung.

2. Verfahren nach Anspruch 1, **dadurch gekennzeichnet, dass** das organische Lösungsmittel aus der Gruppe bestehend aus Acetonitril, N,N-Dimethylformamid, linearem oder verzweigtem C₁-C₄-Alkylalkohol, oder Mischungen davon, ausgewählt ist.

3. Verfahren nach einem der Ansprüche 1 bis 2, **dadurch gekennzeichnet, dass** die Base ein Amin ist, das aus der Gruppe bestehend aus tertiären Aminen, aromatischen Aminen und Mischungen davon, ausgewählt ist.

4. Verfahren nach einem der Ansprüche 1 bis 3, **dadurch gekennzeichnet, dass** die Base Diisopropylethylamin, Triethylamin oder eine Mischung davon, bevorzugt Triethylamin, ist.

5. Verfahren nach einem der Ansprüche 1 bis 4, **dadurch gekennzeichnet, dass** Schritt (a) ferner den Schritt des Erhitzens der Mischung auf eine Temperatur, die zwischen 30 °C und 100 °C umfasst, umfasst.

6. Verfahren nach Anspruch 5, **dadurch gekennzeichnet, dass** der Schritt des Erhitzens der Mischung bei einer Temperatur zwischen 30 °C und 70 °C durchgeführt wird.

7. Verfahren nach einem der Ansprüche 1 bis 6, **dadurch gekennzeichnet, dass** Schritt (c) ferner den Schritt des Erhöhens der Temperatur der in Schritt (b) erhaltenen Mischung zwischen 10 °C und 50 °C umfasst.

8. Verfahren nach einem der Ansprüche 1 bis 7, **dadurch gekennzeichnet, dass** das Rühren von Schritt (c) zwischen 1 und 10 Stunden durchgeführt wird.

9. Verfahren nach einem der Ansprüche 1 bis 8, **dadurch gekennzeichnet, dass** es die zusätzlichen Schritte (d) Erniedrigen der Reaktionstemperatur auf eine Endtemperatur zwischen -5 °C und 10 °C und (e) anschließendes Filtrieren des Produkts der Formel (I), umfasst.

10. Verfahren nach einem der Ansprüche 1 bis 9, **dadurch gekennzeichnet, dass** das Molverhältnis zwischen den Verbindungen der Formel (A) und (B) 1:0,5 bis 1:2, bevorzugt 1:0,95 bis 1:1,5, beträgt.

11. Verfahren nach einem der Ansprüche 1 bis 10, **dadurch gekennzeichnet, dass** das Molverhältnis zwischen der Verbindung der Formel (A) und der Base 1:0,1 bis 1:2,5, bevorzugt 1:0,25 bis 1:2,5, beträgt.

12. Verfahren nach einem der Ansprüche 1 bis 11, **dadurch gekennzeichnet, dass** in Schritt (a) die Massenkonzentration jeder der Verbindungen der Formel (A) und (B) 15 bis 40 % beträgt, ausgedrückt in Gramm gelöster Stoffe pro Milliliter Lösung multipliziert mit 100.

13. Verfahren nach einem der Ansprüche 1 bis 12, **dadurch gekennzeichnet, dass** es den zusätzlichen Schritt des Umwandelns der Verbindung der Formel (I), oder eines Salzes oder Solvats davon, zu N-(5-Chlorpyridin-2-yl)-N'- ((1S, 2R, 4S)-4-(dimethylcarbamoyl)-2-((5-methyl-4,5,6,7-tetrahydrothiazol[5,4-c]pyridin-2-carbonyl)amino)cyclohexyl)oxamid der Formel (II) 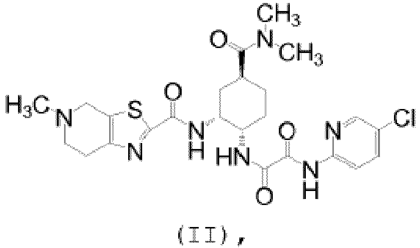 oder eines Salzes oder Solvats davon, umfasst.

## Revendications

1. Procédé pour préparer du N-((1R,2S,5S)-2-((2-((5-chloropyridin-2-yl)amino)-2-oxoacétyl)amino)-5-(diméthylcarbamoyl)cyclohexyl)carbamate de tert-butyle de formule (I) 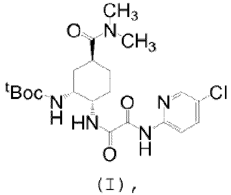 ou un sel ou solvate de celui-ci, **caractérisé en ce qu'**il comporte les étapes consistant à :
a) mélanger le N-((1R,2S,5S)-2-amino-5-(diméthylcarbamoyl)cyclohexyl) carbamate de tert-butyle de formule (A) 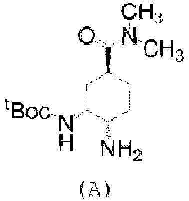 avec du 2-((5-chloropyridin-2-yl)amino)-2-oxoacétate d'éthyle de formule (B) 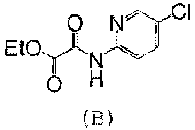 dans un solvant organique,
b) mélanger une base avec le mélange résultant de l'étape (a) ; et
c) agiter le mélange obtenu à l'étape (b).

2. Procédé selon la revendication 1, **caractérisé en ce que** le solvant organique est choisi parmi le groupe constitué d'un acétonitrile, d'un N,N-diméthylformamide, d'un alcool alkyle en C₁ à C₄ linéaire ou ramifié, ou de mélanges de ceux-ci.

3. Procédé selon l'une quelconque des revendications 1 à 2, **caractérisé en ce que** la base est une amine choisie parmi le groupe constitué d'amines tertiaires, d'amines aromatiques et de mélanges de celles-ci.

4. Procédé selon l'une quelconque des revendications 1 à 3, caractérisé ce que la base est une diisopropyléthylamine, une triéthylamine ou un mélange de celles-ci, de préférence une triéthylamine.

5. Procédé selon l'une quelconque des revendications 1 à 4, **caractérisé en ce que** l'étape (a) comporte en outre l'étape consistant à chauffer le mélange à une température comprise entre 30 °C et 100 °C.

6. Procédé selon la revendication 5, **caractérisé en ce que** l'étape de chauffage du mélange est réalisée à une température comprise entre 30 °C et 70 °C.

7. Procédé selon l'une quelconque des revendications 1 à 6, **caractérisé en ce que** l'étape (c) comporte en outre l'étape consistant à augmenter la température du mélange obtenu à l'étape (b) entre 10 °C et 50 °C.

8. Procédé selon l'une quelconque des revendications 1 à 7, **caractérisé en ce que** l'agitation de l'étape (c) est réalisée entre 1 et 10 heures.

9. Procédé selon l'une quelconque des revendications 1 à 8, **caractérisé en ce qu'**il comporte les étapes supplémentaires (d) consistant à réduire la température de réaction jusqu'à une température finale entre -5 °C et 10 °C et (e) filtrer ensuite le produit de formule (I).

10. Procédé selon l'une quelconque des revendications 1 à 9, **caractérisé en ce que** le rapport molaire entre les composés de formules (A) et (B) est de 1:0,5 à 1:2, de préférence de 1:0,95 à 1:1,5.

11. Procédé selon l'une quelconque des revendications 1 à 10, **caractérisé en ce que** le rapport molaire entre le composé de formule (A) et la base est de 1:0,1 à 1:2,5, de préférence de 1:0,25 à 1:2,5.

12. Procédé selon l'une quelconque des revendications 1 à 11, **caractérisé en ce qu'**à l'étape (a), la concentration massique de chacun des composés de formules (A) et (B) est de 15 à 40 %, exprimée en grammes de soluté par millilitre de solution multipliée par 100.

13. Procédé selon l'une quelconque des revendications 1 à 12, **caractérisé en ce qu'**il comporte les étapes supplémentaires consistant à transformer le composé de formule (I), ou un sel ou solvate de celui-ci, en N-(5-chloropyridin-2-yl)-N'-((1S,2R,4S)-4-(diméthylcarbamoyl)-2-((5-méthyl-4,5,6,7-tétrahydrothiazol[5,4-c]pyridin-2-carbonyl)amino)cyclohexyl)oxamide de formule (II) 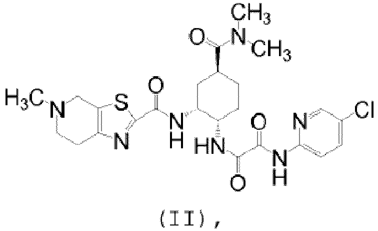 ou un sel ou solvate de celui-ci.
